# EUROPÄISCHE PATENTSCHRIFT

(11) **EP 0 498 333 B1**
(45) Veröffentlichungstag und Bekanntmachung des Hinweises auf die Patenterteilung: **15.01.1997**
(21) Anmeldenummer: 92101708.3
(22) Anmeldetag: 03.02.1992
(51) Int. Cl.: A61K 51/00

(54) **Verfahren zur Herstellung einer mit Technetium-99m markierten organspezifischen Substanz**
Preparation of an organospecific substance marked with technetium-99m
Procédé de préparation de substances organospécifiques marquées au technetium-99m

(30) Priorität: 05.02.1991 DE 4103370
(43) Veröffentlichungstag der Anmeldung: 12.08.1992
(73) Patentinhaber: CIS BIO INTERNATIONAL, F-91000 Saclay (FR)
(72) Erfinder: Kuhlmann, Ludwig, Dr., W-6093 Flörsheim am Main (DE); Mayer, Anton, W-6272 Niedernhausen (DE)
(74) Vertreter: Dubois-Chabert, Guy

(56) Entgegenhaltungen:
- EP-A- 0 028 092
- EP-A- 0 271 806
- EP-A- 0 403 225
- WO-A-89/07456

## Beschreibung

Gegenstand der Erfindung ist ein Verfahren zu Herstellung einer mit Technetium-99m markierten organspezifischen Substanz, die vorbehandelt ist oder mit einem Komplexbildner für Technetium-99m gekoppelt ist, wobei die organspezifische Substanz mit Pertechnetat-99m und einem komplexstabilisierten Reduktionsmittel versetzt wird.

In der medizinischen Diagnostik mit radioaktiven Nukliden werden bereits seit vielen Jahren Proteine erfolgreich eingesetzt. So kann z.B. mit Humanserumalbumin (HSA), markiert mit Technetium-99m oder anderen geeigneten Nukliden, Untersuchungen am Herzen durchgeführt werden.

In der letzten Zeit ist die Bedeutung der Immunglobuline in den Vordergrund getreten, wobei insbesondere monoklonale Antikörper für die Erkennung von bösartigen Veränderungen eingesetzt werden. Monoklonale Antikörper haben sich auch auf anderen Gebieten der nuklearmedizinischen Diagnostik, bewährt, z.B. bei der Lokalisation von Entzündungsherden.

Zunächst wurden die Antikörper mit verschiedenen Iodisotopen (Iod-123 oder Iod-131) oder Indium-111 markiert. Die klinische Prüfung zeigte aber, daß die Verwendung des sehr viel kürzerlebigen Nuklids Technetium-99m (Tc-99m) ebenfalls möglich ist. Dieses Nuklid nimmt in der Nuklearmedizin eine herausragende Stellung ein, da es sehr günstige kernphysikalische Eigenschaften aufweist. Zudem ist es durch den Mo-99/Tc-99m-Generator praktisch zu jeder Zeit und an jedem Ort verfügbar.

Es sind daher eine Reihe von Verfahren beschrieben, in denen Proteine mit Technetium-99m markiert werden. Diese können in zwei große Gruppen unterteilt werden.

In die eine Gruppe sind alle die Verfahren einzuordnen, die zunächst Komplexbildner der unterschiedlichsten Art an den Antikörper koppeln und über diese das Technetium-99m stabil binden. Diese Methode hat jedoch den Nachteil, daß die Markierungsausbeuten nicht hinreichend groß sind und deshalb Reinigungsschritte für die Herstellung des applikationsfertigen Präparates notwendig werden.

Zu der zweiten Gruppe gehört die Methode, bei der das Technetium-99m direkt an den Antikörper gebunden wird. Zu diesem Zweck werden in dem Antikörper reaktive Gruppen erzeugt. Das sind im allgemeinen SH-Funktionen, die durch Reduktion von Disulfidbrücken mit geeigneten Reduktionsmitteln (z.B. 2-Mercaptoethanol, 3-Mercapto-1,2-propandiol, Cystein etc). erzeugt werden. Über diesen Weg markierte Proteine können ohne besondere Reinigungsschritte direkt injiziert werden. Die folgenden Verfahren sind aus dieser Gruppe im einzelnen hervorzuheben.

B.A. Rhodes beschreibt die Markierung eines anti-hCG Antikörper mit Tc-99m (USA-4,472,371). Der Antikörper wird hierbei mit einem Überschuß an Zinn(II)-ionen behandelt, der im Präparat verbleibt. Zinn(II)-ionen bewirken im Antikörper die Reduktion von Disulfidbrücken zu SH-Gruppen sowie andererseits auch die Reduktion von Pertechnetat-99m zu Tc(IV)-99m. In dieser Form ist Tc-99m befähigt, an den Antikörper gebunden zu werden. Wegen des hohen Überschusses an Sn(II)-ionen, tritt Tc-99m nicht nur gebunden am Antikörper auf, sondern es bilden sich auch Tc-Verbindungen, die abgetrennt werden müssen (vgl. US-A-4,472,371 : Beispiel II). Dies ist jedoch ein gravierender Nachteil im Hinblick auf die Bedingungen, die im Labor einzuhalten sind.

In der EP-A-0 271 806 wird eine Markierungsmethode beschrieben, bei der Zinn(II)-ionen in komplexierter Form separat vom Antikörper aufbewahrt werden. Erst kurz vor der Markierung werden die beiden Komponenten zusammengegeben. Die Markierungseinheit besteht daher aus zwei Flaschen. Für den Anwender bedeutet dies einen Nachteil, da er zwei Flaschen handhaben muß, die nicht verwechselt werden dürfen.

Die komplexstabilisierten Sn(II)-ionen werden auch hier als Reduktionsmittel verwendet. Der Komplexbildner wird, bezogen auf Sn(II)-ionen, im Überschuß eingesetzt, d.h. es bilden sich neben Komplexen mit Sn(II)-ionen auch solche mit Tc-99m.

In anderen Schriften des Standes der Technik werden die gebildeten Tc-99m-Komplexe (mit den Komplexbildner des Sn(II)) bewußt erzeugt, um Tc-99m mittels des gebildeten Komplexes auf den Antikörper zu übertragen. Der Vorgang wird als Transkomplexierung bezeichnet:

Gemäß der EP-A-0 237 150 wird ein Tc-99m-Tartrat oder -Glucoheptonat eingesetzt, um einen Antikörper mit Tc-99m zu markieren.

Für den gleichen Zweck werden Tc-99m Komplexe mit Saccharose, Glucoheptonat, Tartrat und Arabonat in der WO 88/07382 verwendet. Der Stabilität dieser Komplexe wird sogar besondere Bedeutung beigemessen.

Auch in der WO 89/07456 wird Tc-99m-Tartrat zur Transkomplexierung verwendet. Hier wird ein Verfahren beschrieben, in dem der Antikörper zunächst an einem Komplexbildner gebunden wird.

Die genannten Verfahren, bei denen Tc-99m durch Transkomplexierung auf den Antikörper übertragen wird, bergen den systembedingten Nachteil, daß infolge einer nicht steuerbaren Umsetzung im fertigen Präparat unerwünschte Tc-99m-Verbindungen enthalten sind.

Es war daher Aufgabe der vorliegenden Erfindung, ein Verfahren zur Herstellung einer mit Technetium-99m markierten organspezifischen Substanz bereitzustellen, bei dem keine unerwünschten Tc-99m-Verbindungen an den Antikörper gebunden sind.

Gelöst wurde die Aufgabe durch Bereitstellung eines Verfahrens der eingangs beschriebenen Gattung, das dadurch gekennzeichnet ist, daß der für das Reduktionsmittel benötigte Komplexbildner in einer, bezogen auf das Reduktionsmittel, stöchiometrischen Menge verwendet wird.

Überraschenderweise wurde - entgegen der bisherigen Vorstellungen - gefunden, daß der Vorgang der Transkomplexierung zur Übertragung von Tc-99m auf den Antikörper nicht notwendig ist.

Es war völlig überraschend zu erkennen, daß der Komplexbildner, lediglich die Aufgabe hat, Sn(II)-ionen in einem Komplex in Lösung zu halten, d.h. ein Ausfällen als Sn(II)-hydroxid zu verhindern. Der bisherige verwendete Überschuß an Komplexbildner, der die Transkomplexierung auslöst, ist nicht notwendig und führt letztendlich auch zu erheblichen Nachteilen: u.a. mußten Sn(II)-ionen und Antikörper in zwei verschiedenen Gefäßen aufbewahrt werden.

Das erfindungsgemäße Verfahren läßt es dagegen zu, daß Sn(II)-Komplex und Antikörper in einem Gefäß aufbewahrt werden können.

Als Reduktionsmittel werden bevorzugt Dithionit oder Sn(II)ionen verwendet.

Unter der "stöchiometrischen" Menge an Komplexbildner ist die Menge zu verstehen, die notwendig ist, um die Valenzen der Sn(II)-ionen vollständig abzusättigen und gleichzeitig aber auch einen Komplex definierter Zusammensetzung zu erreichen.

Die stöchiometrischen Sn(II)-Komplexe sollen in einem pH-Bereich von 3 bis 11, vorzugsweise 4 bis 7 stabil sein.

Das jeweilige stöchiometrische Verhältnis der Sn(II)-ionen zum Komplexbildner ist nicht allgemein festzulegen, da dies vom jeweils gewählten Komplexbildner, abhängt.

Insbesondere werden anionische Komplexe bevorzugt. Hierunter wiederum solche mit einer 4-fach bis 8-fach negativen Ladung. Der Ladungsausgleich wird durch Alkali-, Erdalkali oder NH₄⁺-ionen gewährleistet.

Ganz besonders bevorzugt sind die Komplexe des Sn(II)ions mit Zitronensäure. Der sich bildende stöchiometrische anionische Sn(II)-Zitronensäurekomplex [Sn(Citrat)₂]⁴⁻ ist bekannt (Gmelin, Handbuch der Anorganischen Chemie, Zinn, Teil C, S. 217-229, Heidelberg 1975).

Dagegen ist der ebenso besonders bevorzugte Komplex des Sn(II)-ions mit 1,1,3,3-Propan-tetraphosphonsäure bisher nicht beschrieben. Er ist aber für den Fachmann durch bekannte Methoden zugänglich, bei dem 1,1,3,3-Propantetraphosphonsäure zu Sn(II) im molaren Verhältnis von 2:1 miteinander reagiert und einen Komplex bildet, der in seiner Zusammensetzung diesem molaren Verhältnis entspricht. (s.u.).
Dagegen würde ein Unterschreiten des genannten molaren Verhältnisses zur Ausbildung einer schwerlöslichen Verbindung führen, in der ein Molekül Phosphonsäure 4 Sn(II)-ionen bindet.

Organspezifische Substanzen nach dem erfindungsgemäßen Verfahren sind im allgemeinen Trägersubstanzen, die in ihrem Molekül mindestens eine funktionelle Gruppe mit komplexierenden Eigenschaften besitzen. Meist handelt es sich bei solchen Gruppen um Atome oder Ionen mit Elektronenpaardonorfunktion (Lewis-Basen). Ein solche funktionelle Gruppe mit komplexierender Eigenschaft ist beispielsweise eine -SCN-, -NH₂-, -NHR-, -NR₂-, -COO-, -OH-, =S-, -SH-, -NO-Gruppe.

Als Vertreter für solche Substanzen mit funktionellen, komplexierenden Gruppen seien beispielsweise genannt: Proteine (-NH-, -NH₂- oder COO-Gruppen), Enzyme (-NH₂-, -OH-, -P= O-Gruppen), Zucker (-OH-Gruppen) oder Polymere, welche Seitenketten mit entsprechenden funktionellen Gruppen aufweisen.

Weist die zu markierende Verbindung eine solche funktionelle Gruppe nicht auf, so muß vor der Markierung die Substanz "vorbehandelt" oder an einen geeigneten Komplexbildner gekoppelt werden.

Unter "vorbehandelt" werden im Sinne der Erfindung solche Maßnahmen verstanden, die dazu führen, daß eine funktionelle Gruppe mit komplexbildenden Eigenschaften im zu markierenden Molekül entstehen. Beispielsweise enthalten Antikörper Disulfid-Brücken. Die beiden kovalent miteinander verknüpften Schwefelatome sind jedoch in dieser Form nicht befähigt, Technetium-99m zu komplexieren. Reduziert man jedoch die Disulfid-Brücke, so entstehen zwei SH-Gruppen, die nun ihrerseits ausgezeichnete Komplexliganden für Technetium-99m darstellen und dieses auch in guten Ausbeuten binden.

Eine andere Möglichkeit, Technetium-99m an organspezifische Substanzen, die keine funktionelle Gruppe mit komplexierenden Eigenschaften besitzt, zu binden, besteht darin, eine solche funktionelle Gruppe in das Molekül einzubauen oder ein Komplexierungsagenz an das Molekül chemisch zu binden.

Besonders interessant erscheint das Verfahren für die Technetium-99m-Markierung von Antikörpern. Eine partielle Reduktion der S-S-Bindungen des Antikörpers oder eines F(ab')₂-Antikörperfragments läßt sich bei Raumtemperatur durch kurzzeitige Einwirkung von milden Reduktionsmitteln erreichen (Vorbehandeln der organspezifischen Substanz). Besonders geeignete Reduktionsmittel sind Monothiole wie 2-Mercaptoethanol oder 2-Mercaptoethylamin (Cysteamin). Dabei erhält man reaktionsfähige Antikörpermoleküle, die weder ihre immunologische Reaktivität eingebüßt haben noch zu kleineren Bruchstücken fragmentiert worden sind. Grundsätzlich sind für die partielle Reduktion des Antikörpers oder des F(ab')₂-Antikörperfragments alle Reduktionsmittel geeignet, die auch bei längerer Einwirkungszeit nur einen Teil der S-S-Bindungen spalten und zu keiner Fragmentierung der Antikörperkomponente führen. Die Einwirkungszeit eines derartigen Reduktionsmittels auf die Antikörperkomponente braucht eine Stunde nicht zu übersteigen. Im allgemeinen sind schon nach 10 bis 30 Minuten so viele SH-Gruppen entstanden, daß ausreichende Mengen von Technetium-99m-Kationen gebunden werden. Dann wird das überschüssige Reduktionsmittel abgetrennt und der partiell reduzierte Antikörper in einer gepufferten Lösung (z.B. 0,02 M Phosphatlösung, pH 7,2) vereinzelt und umgehend lyophilisiert. Dabei muß eine Reoxidation der freien Thiolgruppen im Antikörper durch Luft-Sauerstoff unterbunden werden. Der lyophilisierte Antikörper, der außer den Puffersalzen keine weiteren Zusätze enthält und mit Stickstoff als Schutzgas überlagert wird, ist bei Kühlschranktemperatur (-5 bis +5 °C) wochenlang unverändert haltbar; er löst sich bei Zugabe von isotonischer Natriumchloridlösung einwandfrei wieder auf.

Die so hergestellte, partiell reduzierte Antikörperkomponente (vorbehandelte organspezifische Substanz) kann nun glatt nach dem erfindungsgemäßen Verfahren mit Tc-99m markiert werden, wenn man ihr ein Gemisch aus Pertechnetat und stöchiometrischen Sn(II)-Komplex zusetzt.

Zur Herstellung eines einsatzbereiten Diagnostikums kann man nun so vorgehen, daß man zuerst die lyophilisierte Antikörperkomponente in einer Technetium-99m-Pertechnetatlösung auflöst und dann die Reduktion und Bindung des Technetiums an den Antikörper durch Zugabe einer Lösung des Zinn-(II)-Komplexes bewirkt.

Man kann das Diagnostikum aber auch dadurch herstellen, daß man die Antikörperkomponente zuerst in der Zinn-(II)-komplex-haltigen Lösung auflöst und anschließend durch Zugabe von Technetium-99m-Pertechnetatlösung die lyophilisierte Antikörperkomponente mit Technetium markiert.

Eine weitere besondere Ausführungsart der Erfindung ist im Patentanspruch 12 angegeben.

Zur Herstellung eines Diagnostikums, welches eine Technetium-99m-markierte organspezifische Substanz enthält, wird zweckmäßigerweise ein Test zusammengestellt, der die organspezifische Substanz bzw. die vorbehandelte organspezifische Substanz oder die mit einem Komplexbildner für Tc-99m gekoppelte, organspezifische Substanz, ggf. im Gemisch mit einem Puffer und das komplexstabilisierte Zinn-(II)-Salz enthält, das zur Reduktion des Technetiums an der organspezifischen Substanz benötigt wird. Besonders bewährt hat sich ein Test, bei dem die lyophilisierte, gegebenenfalls vorbehandelte organspezifische Substanz im Gemisch mit Dinatriumhydrogenphosphat (pH 7,2) als Puffersubstanz vorliegt. Man erhält so nach kurzer Reaktionszeit, z.B. schon nach 5 Minuten, eine nahezu quantitative Technetium-99m-Markierung der Substanz, die weniger als 1 % freies Pertechnetat und nur sehr geringe Mengen der Tc-99m-markierten Zinn-II-Komponente als Verunreinigungen enthält, so daß anschließende Reinigungsverfahren nicht mehr erforderlich sind.

Die gemäß dem erfindungsgemäßen Verfahren hergestellte organspezifische Substanz gewährleistet - obwohl sie in einem Gefäß mit dem Sn(II)-Komplex aufbewahrt wird - eine unveränderte Haltbarkeit des lyophilisierten Präparats. Damit ist eine schnelle, unproblematische und einwandfreie Markierung der organspezifischen Substanz gewährleistet.

Der in manchen Markierungsbestecken vorhandene Stabilisator ist auch bei der Antikörpermarkierung von Vorteil, da er eine noch längere Haltbarkeit der Injektionslösung garantiert.

Die folgenden Beispiele sollen die Erfindung erläutern:

### Beispiel 1: Herstellung eines stabilen Komplexes zwischen Zinn(II) und 1,1,3,3-Propantetraphosphonsäure

0,5240 g (1,0 mmol) 1,1,3,3-Propantetraphosphonsäure, -tetranatriumsalz, -tetrahydrat (PTP) werden in 100 ml Wasser gelöst. 0,2257 g (1,0 mmol) Zinn(II)-dichlorid, -dihydrat werden in 100 ml 0,1 n Salzsäure gelöst. In einem Becherglas werden 2 ml (0,02 mmol der PTP-Lösung und 1 ml (0,01 mmol)) der Zinn(II)-Lösung miteinander vermischt und der pH-Wert mit 2 n Natronlauge auf einen gewünschten Wert zwischen pH = 3 bis 11 eingestellt. Es wird eine klare Lösung erhalten.

Wird 1 ml (0,01 mmol) der PTP-Lösung und 1 ml (0,01 mmol) der Zinn(II)-Lösung miteinander vermischt, so entsteht sofort ein Niederschlag der auch durch Änderungen des pH-Wertes im Bereich von pH = 3 bis 11 nicht aufgelöst werden kann. In dem Überstand können keine Zinn(II)-ionen aber noch 3 Äquivalente an PTP nachgewiesen werden.

In dem Beispiel 2 wird die Herstellung des Zinn(II)-citrat-Komplexes beschrieben.

### Beispiel 2: Herstellung eines Zinn(II)-Komplexes mit Citronensäure

0,12955 g Citronensäure, wasserfrei und 0,07603 g Zinn(II)-dichlorid, -dihydrat werden in 10 ml Wasser gelöst. Die Lösung wird mit Wasser auf 900 ml verdünnt, der pH-Wert mit 2 n Natornlauge auf pH = 6,5 bis 7 eingestellt und danach das Volumen mit Wasser auf genau 1 l aufgefüllt. Die Lösung enthält 40 µg Sn(II)/ml und kann direkt für die Herstellung der Antikörperpräparate verwendet werden.

Das folgende Beispiel zeigt die Herstellung eines Markierungbestecks mit dem monoklonalen Antikörper BW 494/32. Dieser Antikörper reagiert mit Antigenen, die vorwiegend von Mamma- oder Ovarcarcinomzellen exprimiert werden.

### Beispiel 3: Herstellung einer Markierungseinheit mit dem monoklonalen Antikörper BW 494/32

Zu 1 ml eine Lösung des mit 2-Mercaptoethanol, 3-Mercapto-1,2-propandiol oder mit einem anderen geeigneten Reduktionsmittel behandelten Antikörpers mit 1 mg an Immunglobulin werden 0,5 ml der Lösung aus Beispiel 1 oder Beispiel 2 gegeben. Die Lösungen werden miteinander vermischt und danach gefriergetrocknet.

Zur Markierung mit Tc-99m wird wie folgt vorgegangen.

Zu dem Lyophilisat gibt man das Eluat eines handelsüblichen Generators mit einer Aktivität von 500 MBq bis 1500 MBq. Diese Aktivität kann sich in einem Volumen von 1 ml bis 10 ml befinden. Nach einer Wartezeit von 5 min bis 10 min ist das Präparat injektionsfertig.

Die Überprüfung der radiochemischen Reinheit mit der Dünnschichtchromatographie (ITLC SG/Methylethylketon) oder Hochleistungs-Flüssigkeitschromatographie (Gelfitrationsäule, (z.B. Bio Rad TSK 250), 0,1 m Phosphatpuffer pH 6,8, Fluß: 1 ml/min) ergab, das zwischen 95 % und 99 % der zugegebenen Aktivität an den Antikörper gebunden waren. Bis zu einer Wartezeit von 24 Stunden blieb diese Lösung ausreichend stabil.

Im Tierversuch an tumortragenden Nacktmäusen wurden mit diesen Präparaten Speicherungen im Tumor zwischen 6 und 7 %/g Tumor erzielt.

## Patentansprüche

1. Verfahren zur Herstellung einer mit Technetium-99m markierten organspezifischen Substanz, die in der Weise vorbehandelt ist, daß eine funktionelle Gruppe mit komplexbildenden Eigenschaften entsteht , oder mit einem Komplexbildner für Technetium-99m gekoppelt ist, wobei die organspezifische Substanz mit Pertechnetat-99m und einem komplexstabilisierten Reduktionsmittel versetzt wird, dadurch gekennzeichnet, daß der für das Reduktionsmittel benötigte Komplexbildner in einer für das Reduktionsmittel stöchiometrischen Menge verwendet wird.

2. Verfahren nach Anspruch 1, dadurch gekennzeichnet, daß das Einsetzen einer stöchiometrischen Menge zu einem Komplex definierter Zusammensetzung führt.

3. Verfahren nach den Ansprüchen 1 oder 2, dadurch gekennzeichnet, daß der Komplex des Reduktionsmittels in einem pH-Bereich von 3 bis 11 stabil ist.

4. Verfahren nach einem oder mehreren der Ansprüche 1 bis 3, dadurch gekennzeichnet, daß der Komplex ein anionischer Komplex ist.

5. Verfahren nach einem oder mehreren der Ansprüche 1 bis 4, dadurch gekennzeichnet, daß als Reduktionsmittel Sn(II)-ionen verwendet werden.

6. Verfahren nach einem oder mehreren der Ansprüche 1 bis 5, dadurch gekennzeichnet, daß als Komplexbildner Zitronensäure und/oder 1,1,3,3-Propantetraphosphonsäure verwendet werden.

7. Verfahren nach einem oder mehreren der Ansprüche 1 bis 6, dadurch gekennzeichnet, daß als organspezifische Substanzen Proteine, Enzyme, Zucker oder Polymere verwendet werden.

8. Verfahren nach den Ansprüche 1 und 7, dadurch gekennzeichnet, daß man einen monoklonalen Antikörper oder dessen F(ab')₂-Fragment einsetzt.

9. Verfahren nach Anspruch 8, dadurch gekennzeichnet, daß man einen Antikörper oder dessen F(ab')₂-Fragment gegen Tumor assoziierte Antigene einsetzt.

10. Diagnostikum, dadurch hergestellt, daß man zuerst die Komponente, die die organspezifische Substanz enthält, in einer Technetium-99m-Pertechnetat-Lösung auflöst und dann die Reduktion und Bindung des Technetiums an die organspezifische Substanz durch Zugabe eines komplexstabilisierten Reduktionsmittels bewirkt.

11. Diagnostikum, dadurch hergestellt, daß man die Komponente, die die organspezifische Substanz enthält, zuerst in der Lösung des komplexstabilisierten Reduktionsmittels auflöst und anschließend durch Zugabe von Technetium-99m-Pertechnetat-Lösung die organspezifische Substanz mit Technetium markiert.

12. Diagnostium nach den Ansprüche 10 und 11, dadurch gekennzeichnet, daß das komplexstabilisierte Reduktionsmittel ein stöchiometrischer Komplex des Sn(II) mit Zitronensäure oder mit 1,1,3,3-Propantetraphosphonsäure ist, wobei von diesem Reduktionsmittel, bezogen auf Sn(II), jeweils 1 bis 100 µg, vorzugsweise 5 bis 10 µg pro 1 mg der organspezifischen Substanz zugesetzt werden, um diese stabil mit Technetium-99m zu markieren.

## Claims

1. Process for the preparation of an organ-specific substance which is labelled with technetium-99m and which has been pretreated in such a way that a functional group with complex-forming properties results or coupled to a complexing agent for technetium-99m, where the organ-specific substance is mixed with pertechnetate-99m and a complex-stabilized reducing agent, characterized in that the complexing agent which is required for the reducing agent is used in an amount which is stoichiometric for the reducing agent.

2. Process according to Claim 1, characterized in that the employment of a stoichiometric amount leads to a complex of defined composition.

3. Process according to Claims 1 or 2, characterized in that the complex of the reducing agent is stable in a pH range from 3 to 11.

4. Process according to one or more of Claims 1 to 3, characterized in that the complex is an anionic complex.

5. Process according to one or more of Claims 1 to 4, characterized in that Sn(II)ions are used as reducing agent.

6. Process according to one or more of Claims 1 to 5, characterized in that citric acid and/or 1,1,3,3-propanetetra-phosphonic acid are used as complexing agent.

7. Process according to one or more of Claims 1 to 6, characterized in that proteins, enzymes, sugars or polymers are used as organ-specific substances.

8. Process according to Claims 1 and 7, characterized in that a monoclonal antibody or its F(ab')₂ fragment is employed.

9. Process according to Claim 8, characterized in that an antibody or its F(ab')₂ fragment against tumour-associated antigens is employed.

10. Diagnostic aid prepared by initially dissolving the component which contains the organ-specific substance in a technetium-99m-pertechnetate solution, and then bringing about the reduction and binding of the technetium to the organ-specific substance by adding a complex-stabilized reducing agent.

11. Diagnostic aid prepared by initially dissolving the component which contains the organ-specific substance in the solution of the complex-stabilized reducing agent, and subsequently labelling the organ-specific substance with technetium by adding technetium-99m pertechnetate solution.

12. Diagnostic aid according to Claims 10 and 11, characterized in that the complex-stabilized reducing agent is a stoichiometric complex of Sn(II) with citric acid or with 1,1,3,3-propanetetraphosphonic acid, adding in each case 1 to 100 µg, preferably 5 to 10 µg, based on Sn(II), of this reducing agent per 1 mg of the organ-specific substance for stable labelling of the latter with technetium-99m.

## Revendications

1. Procédé de préparation d'une substance organospécifique marquée au technétium 99m qui est prétraitée de manière à former un groupe fonctionnel ayant des propriétés complexantes, ou bien couplée à un agent complexant pour le technétium 99m, dans lequel la substance organospécifique est mélangée avec du pertechnétate 99m et un agent réducteur à complexe stabilisé, caractérisé en ce que l'agent complexant nécessaire pour l'agent réducteur est utilisé en une quantité stoechiométrique pour l'agent réducteur.

2. Procédé selon la revendication 1, caractérisé en ce que l'utilisation d'un quantité stoechiométrique donne un complexe ayant une composition définie.

3. Procédé selon les revendications 1 ou 2, caractérisé en ce que le complexe de l'agent réducteur est stable dans une plage de pH de 3 à 11.

4. Procédé selon l'une ou plusieurs des revendications 1 à 3, caractérisé en ce que le complexe est un complexe anionique.

5. Procédé selon l'une ou plusieurs des revendications 1 à 4, caractérisé en ce que des ions stanneux Sn(II) sont utilisés comme agent réducteur.

6. Procédé selon l'une ou plusieurs des revendications 1 à 5, caractérisé en ce que l'acide citrique et/ou l'acide 1,1,3,3-propane-tétraphosphonique sont utilisés comme agent complexant.

7. Procédé selon l'une ou plusieurs des revendications 1 à 6, caractérisé en ce que des protéines, des enzymes, des sucres ou des polymères sont utilisés comme substances organospécifiques.

8. Procédé selon les revendications 1 et 7, caractérisé en ce que l'on utilise un anticorps monoclonal ou le fragment F(ab')₂ de celui-ci.

9. Procédé selon la revendication 8, caractérisé en ce que l'on utilise un anticorps dirigé contre des antigènes associés aux tumeurs ou le fragment F(ab')₂ de celui-ci.

10. Produit de diagnostic préparé en dissolvant d'abord le composant contenant la substance organospécifique dans une solution de technétium 99m-pertechnétate puis en réalisant la réduction et la liaison du technétium à la substance organospécifique par addition d'un agent réducteur à complexe stabilisé.

11. Produit de diagnostic préparé en dissolvant d'abord le composant contenant la substance organospécifique dans une solution de l'agent réducteur à complexe stabilisé, puis en marquant la substance organospécifique au technétium par addition de solution de technétium 99m-pertechnétate.

12. Produit de diagnostic selon les revendications 10 et 11, caractérisé en ce que l'agent réducteur à complexe stabilisé est un complexe stoechiométrique de Sn(II) avec l'acide citrique ou l'acide 1,1,3,3-propane-tétraphosphonique, ledit agent réducteur étant ajouté à raison de 1 à 100 µg, de préférence 5 à 10 µg, par rapport au Sn(II), pour 1 mg de substance organospécifique afin de marquer cette dernière de façon stable au technétium 99m.
